# EUROPÄISCHE PATENTSCHRIFT

(11) **EP 1 512 681 B1**
(45) Veröffentlichungstag und Bekanntmachung des Hinweises auf die Patenterteilung: **23.04.2008**
(21) Anmeldenummer: 04103131.1
(22) Anmeldetag: 02.07.2004
(51) Int. Cl.: C07C 263/04, C07C 263/06, C07C 265/14

(54) **Mehrstufiges Verfahren zur kontinuierlichen Herstellung von cycloaliphatischen Diisocyanaten**
Multi-step process for the continuous preparation of cycloaliphatic diisocyanates
Procédé à plusieurs étapes pour la préparation continue de diisocyanates cycloaliphatiques

(30) Priorität: 22.08.2003 DE 10338508
(43) Veröffentlichungstag der Anmeldung: 09.03.2005
(73) Patentinhaber: Evonik Degussa GmbH, 40474 Düsseldorf (DE)
(72) Erfinder: Kohlstruk, Stephan, Dr., 48249 Dülmen (DE); Kreczinski, Manfred, 44652 Herne (DE); Elm, Rainer, Dr., 45772 Marl (DE); Michalczak, Hans-Werner, 44651 Herne (DE)

(56) Entgegenhaltungen:
- EP-A- 0 355 443
- EP-A- 0 566 925
- EP-A- 0 568 782

## Beschreibung

Die Erfindung betrifft ein Verfahren zur kontinuierlichen phosgenfreien Herstellung von cycloaliphatischen Diisocyanaten.

Der synthetische Zugang zu Isocyanaten kann über eine Reihe unterschiedlicher Routen erfolgen. Älteste und auch heute noch vorherrschende Variante zur großtechnischen Herstellung von Isocyanaten ist die so genannte Phosgenroute. Grundlage dieses Verfahrens ist Umsetzung von Aminen mit Phosgen. Nachteil des Phosgenverfahrens ist der Einsatz von Phosgen, dass aufgrund seiner Toxizität und Korrosivität besonders hohe Anforderungen an seine Handhabung im industriellen Maßstab stellt.

Es gibt mehrere Verfahren, die Verwendung von Phosgen zur Herstellung von Isocyanaten in technischen Größenordnungen zu umgehen. Der Begriff phosgenfreies Verfahren wird häufig im Zusammenhang mit der Überführung von Aminen in Isocyanate unter Einsatz alternativer Carbonylierungsmittel, z. B. Harnstoff oder Dialkylcarbonat, benutzt (EP 0 018 586, EP 0 355 443, US 4,268,683, EP 0 990 644).

Grundlage der so genannten Harnstoffroute ist die Harnstoff-vermittelte Überführung von Diaminen in Diisocyanate über einen zweistufigen Prozess. Im ersten Schritt wird ein Diamin mit Alkohol in Gegenwart von Harnstoff oder Harnstoff-Äquivalenten (z. B. Alkylcarbonate, Alkylcarbamate) zu einem Diurethan umgesetzt, welches üblicherweise eine Zwischenreinigungstufe durchläuft und dann im zweiten Schritt thermisch in Diisocyanat und Alkohol gespalten wird (EP 0 355 443, US 4,713,476, US 5,386,053). Alternativ kann der eigentlichen Urethanbildung auch die separate Herstellung eines Diharnstoffs durch gezielte Umsetzung des Diamins mit Harnstoff vorgeschaltet sein (EP 0 568 782). Denkbar ist auch eine zweistufige Sequenz aus partieller Umsetzung von Harnstoff mit Alkohol im ersten und anschließender Zudosierung und Urethansierung des Diamins im zweiten Schritt (EP 0 657 420).

Die thermische Spaltung von Urethanen in die entsprechenden Isocyanate und Alkohole ist seit langem bekannt und kann sowohl in der Gasphase bei hohen Temperaturen als auch bei relativ niedrigen Temperaturen in der Flüssigphase durchgeführt werden. Problematisch ist jedoch bei beiden Verfahrensweisen, dass durch die thermische Belastung grundsätzlich auch unerwünschte Nebenreaktionen stattfinden, die zum einen die Ausbeute mindern und zum anderen zur Bildung verharzender Nebenprodukte führen, die den Ablauf eines technischen Prozesses durch Belegungen und Verstopfungen in Reaktoren und Aufarbeitungsvorrichtungen erheblich stören.

Es hat daher nicht an Vorschlägen gefehlt, durch chemische und verfahrenstechnische Maßnahmen Ausbeuteverbesserungen zu erzielen und die unerwünschte Nebenproduktbildung einzuschränken. So wird in einer Reihe von Dokumenten der Einsatz von Katalysatoren beschrieben, die die Spaltreaktion der Urethane beschleunigen (DE 10 22 222, US 3,919,279, DE 26 35 490). Tatsächlich gelingt es in Gegenwart geeigneter Katalysatoren - hierbei handelt es sich um eine Vielzahl basischer, saurer sowie metallorgansicher Verbindungen - durchaus, die Isocyanatausbeute im Vergleich zur unkatalysierten Variante zu steigern. Die Bildung unerwünschter Nebenprodukte kann jedoch auch durch die Anwesenheit eines Katalysators nicht vermieden werden. Dasselbe gilt für die zusätzliche Verwendung von inerten Lösemitteln, wie sie ebenfalls in der US 3,919,279 und DE 26 35 490 empfohlen werden, um eine gleichmäßige Verteilung der zugeführten Wärme und des Katalysators im Reaktionsmedium zu gewährleisten. Grundsätzlich hat die Verwendung von unter Rückfluss siedenden Lösemitteln jedoch eine Reduzierung der Raum/Zeit-Ausbeute an Isocyanaten zur Folge und ist darüber hinaus mit dem Nachteil eines zusätzlichen hohen Energieaufwands behaftet.

In der EP 0 054 817 angeführte Beispiele zur thermisch geführten katalysierten Spaltung von Monourethanen beschreiben die Teilausschleusung des Reaktionsgemisches zur Abtrennung der im Zuge der Urethanspaltung entstehenden verharzenden Nebenprodukte. Diese Prozedur dient der Vermeidung von Belegungen und Verstopfungen in Reaktoren und Aufarbeitungseinrichtungen. Hinweise, die auf eine Ausbeute steigernde Verwertung der Teilausschleusung hindeuten, gibt es nicht. Die EP 0 061 013 beschreibt einen ähnlichen Lösungsansatz, wobei die Thermolyse in diesem Fall in Gegenwart von Lösemitteln durchgeführt wird, deren Aufgabe offenbar in einer besseren Aufnahme der schwerflüchtigen Nebenprodukten besteht. Auch hier wird die Teilausschleusung nicht im Sinne einer Ausbeuteoptimierung verwertet.

Aus der EP 0 355 443 ist nun bekannt, dass eine Ausbeutesteigerung erzielt werden kann, wenn die während der Spaltung von Diurethanen im Spaltreaktor entstehenden höhermolekularen, verwertbaren und nicht verwertbaren Nebenprodukte zur Gewährleistung einer störungsfreien und selektiven Reaktion möglichst kontinuierlich aus dem Reaktor ausgeschleust werden und anschließend in Gegenwart von Alkohol zum großen Teil umgesetzt und dann in die Diurethanherstellung zurückgeführt werden. Die beschriebene Verfahrensweise ist mit einem hohen Energieaufwand verbunden, da die Abtrennung nicht verwertbarer Nebenprodukte aus dem Austrag der Diurethanherstellung destillativ erfolgt, wobei das gesamte Diurethan verdampft werden muss. Im Unterschied zur EP 0 355 443 wird der Urethanisierungsaustrag beim Verfahren der EP 0 566 925 in zwei Teilströme aufgeteilt, von denen nur einer destillativ von seinen hochsiedenden, nicht verwertbaren Nebenprodukten befreit wird, bevor die vereinigten Diurethanströme der Deblockierungsreaktion im Spaltreaktor zugeführt werden. Zudem wird die kontinuierliche Spaltreaktorausschleusung bei der EP 0 566 925 direkt, d.h. ohne Reurethanisierungsschritt, in die Diurethansynthese zurückgeführt.

Es hat sich jedoch herausgestellt, dass die Verfahren der EP 0 355 443 und EP 0 566 925 bei Einsatz von cycloaliphatischen Diaminen von einer deutlich erkennbaren Abnahme der Selektivität betroffen sind, wenn der kontinuierliche Prozess über einen Zeitraum von mehreren Stunden aufrechterhalten wird.

Aufgabe der vorliegenden Erfindung war es daher, ein Verfahren zur kontinuierlichen Herstellung von cycloaliphatischen Diisocyanaten zur Verfügung zu stellen, das gute Ausbeuten liefert und nicht mit dem dargestellten Nachteil einer deutlich erkennbaren Abnahme der Selektivität bei mehrstündigem Betrieb behaftet ist.

Gelöst wurde die Aufgabe durch das Finden eines kontinuierlichen Verfahrens, wobei die Diurethane nach ihrer Synthese durch Umsetzung von cycloaliphatischen Diaminen mit Alkohol und einem geeignetem Carboxylierungsmittel wie Harnstoff und/oder Harnstoff-Äquivalenten von Leicht-, Mittel- und Hochsiedern befreit werden, das so aufgereinigte cycloaliphatische Diurethan unter Freisetzung des gewünschten cycloaliphatichen Diisocyanats thermisch gespalten wird, ein Teil des Spaltsumpfes der Spaltapparatur kontinuierlich ausgeschleust und mit Alkohol reurethanisiert wird und die Rückführung des Reurethanisatstroms direkt in die Leichtsiederabtrennung erfolgt.

Gegenstand der Erfindung ist ein mehrstufiges Verfahren zur kontinuierlichen Herstellung von cycloaliphatischen Diisocyanaten, durch Umsetzung von cycloalipahtischen Diaminen mit Kohlensäurederivaten und Alkoholen zu cycloaliphatischen Diurethanen und anschließenden thermischen Spaltung der Diurethane zu cycloaliphatischen Diisocyanten, dadurch gekennzeichnet, dass die Diurethane nach ihrer Synthese durch Umsetzung von cycloaliphatischen Diaminen mit Alkohol und Harnstoff und/oder Harnstoffderivaten von Leicht-, Mittel- und Hochsiedern befreit werden, das so aufgereinigte cycloaliphatische Diurethan unter Freisetzung des gewünschten cycloaliphatischen Diisocyanats thermisch gespalten wird, ein Teil des Spaltsumpfes der Spaltapparatur kontinuierlich ausgeschleust und mit Alkohol reurethanisiert wird und die Rückführung des Reurethanisatstroms direkt in die Leichtsiederabtrennung erfolgt.

Gegenstand der Erfindung ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Harnstoff und/oder Harnstoffderivaten und Alkoholen in cycloaliphatische Diurethane, und deren thermische Spaltung, wobei
a) cycloaliphatische Diamine der Formel (II)

   H₂N-R-NH₂

   wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)

   R¹-OH

   wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren zu cycloaliphatischen Diurethanen, umgesetzt werden und das entstehende Ammoniak gleichzeitig abtrennt wird;
b) aus der erhaltenen Reaktionsmischung der Alkohol, die Dialkylcarbonate und/oder Carbamidsäurealkylester abtrennt werden und der Alkohol sowie optional die Dialkylcarbonate und/oder Carbamidsäurealkylester in die Reaktionsstufe a) zurückführt werden;
c) der im wesentlichen Diurethane enthaltende Stoffstrom aus Stufe b) destillativ in einen Wertstoffstrom und einen Nebenproduktstrom, der ausgeschleust wird, getrennt wird;
d) die über die Schritte b) und c) aufgereinigten Diurethane enthaltende Reaktionsmischung in Gegenwart eines Katalysators lösemittelfrei bei Temperaturen von 180 - 280 °C, vorzugsweise 200 - 260 °C, und unter einem Druck von 0,1 - 200 mbar, vorzugsweise 0,2 - 100 mbar kontinuierlich thermisch so gespalten wird, dass ein Teil des Reaktionsgemisches von 10 - 60 Gew.-% bezogen auf den Feed, vorzugsweise 15 - 45 Gew.-% bezogen auf den Feed, ständig ausgeschleust wird;
e) die Spaltprodukte durch Rektifikation in ein rohes cycloaliphatisches Diisocyanat und Alkohol getrennt werden;
f) das rohe cycloaliphatische Diisocyanat, durch Destillation gereinigt und die Reinproduktfraktion isoliert wird;
g) die Sumpfausschleusung aus d) mit dem Alkohol aus e) in Gegenwart oder Abwesenheit von Katalysatoren innerhalb von 1 - 150 min, vorzugsweise 3 - 60 min, bei Temperaturen von 20 - 200 °C, vorzugsweise 50 - 170 °C und bei einem Druck von 0,5 - 20 bar, vorzugsweise 1-15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;
h) ein Teil der Sumpffraktion der Reindestillation f) kontinuierlich ausschleust und in die Spaltreaktion d), vorzugsweise aber die Urethanisierungsstufe g) geführt wird;
i) die bei der Reindestillation des cycloaliphatischen Diisocyanats anfallende Kopffraktion ebenfalls in die Urethanisierungsstufe g) zurückgeführt oder verworfen wird;
j) der Reurethanisatstrom aus g) in Stufe b) zurückgeführt wird,
   oder
k) der Reurethanisatstrom aus g) in die Reaktionsstufe a) zurückgeführt wird, unter der Voraussetzung, das g) in Gegenwart von Katalysatoren bevorzugt ausgewählt aus den Halogeniden von Fe(III) und/oder Cu(I), durchgeführt wird.

Nach dem erfindungsgemäßen Verfahren können cycloaliphatische Diisocyanate im kontinuierlichen Betrieb problemlos mit sehr guten Selektivitäten hergestellt werden. Vorteilhaft bei dem erfindungsgemäßen Mehrstufenverfahren ist insbesondere die Tatsache, dass auch bei Einsatz von cycloaliphatischen Diaminen der Formel (II) als Ausgangsmaterial für die kontinuierliche Diisocyanatsynthese über einen langen Zeitraum eine mit hoher Selektivität störungsfrei ablaufende Reaktion gewährleistet wird.
a) Zur Herstellung der monomeren cycloaliphatischen Diurethane in der Reaktionsstufe a) werden die cycloaliphatischen Diamine der Formel (II), mit Harnstoff und/oder als Carboxylierungsmittel geeigneten Harnstoffderivaten und einem Alkohol der Formel (III), ggf. auch Mischungen solcher Alkohole, in einem Molverhältnis von 1: 2,01 : 4,0 bis 1: 2,2 : 10, vorzugsweise 1 : 2,02 : 6 bis 1 : 2,12 : 9, ggf. aber nicht bevorzugt in Gegenwart von Dialkycarbonaten, Carbamidsäurealkylestern oder Mischungen aus Dialkylcarbonaten und Carbamidsäureestern in einer Menge von jeweils 1 - 10 Mol% bezogen auf das das Diamin, in Abwesenheit oder Gegenwart von Katalysatoren bei Reaktionstemperaturen von 140 - 270 °C, vorzugsweise 160 - 250 °C und unter einem Druck, der in Abhängigkeit vom eingesetzten Alkohol zwischen 2 - 80 bar, vorzugsweise 7 - 15 bar beträgt, innerhalb von 2 bis 20 Stunden, vorzugsweise 4 - 9 Stunden, zur Reaktion gebracht. Die Umsetzung kann in einer kontinuierlich betriebenen Rührkesselkaskade, vorzugsweise aber in einem Druckdestillationsreaktor, erfolgen.
   Zur Erhöhung der Reaktionsgeschwindigkeit können die Diurethane, in Gegenwart von Katalysatoren hergestellt werden. Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14^{th} Edition, publiziert von Chemical Rubber Publishing Co. 2310 Superior Ave. N.E. Cleveland, Ohio, enthalten, beispielsweise Halogenide wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocaramate. Beispielhaft genannt seinen die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Kobalt und Nickel. Als typische Katalysatoren seinen beispielhaft folgende Verbindungen genannt: Lithiumethanolat, Lithiumbutanolat, Natriummethanolat, Kallum-tert.-butanolat, Magnesiumethanolat, Calciummethanolat, Zinn-(II)-chlorid, Zinn-(IV)-chlorid, Bleiacetat, Aluminiumtrichlorid, Bismuttrichlorid, Kupfer-(II)-acetat, Kupfer-(II)-chlorid, Zinkchlorid, Zinkoctoat, Titantetrabutanolat, Vanadiumtrichlorid, Vanadiumacetylacetonat, Mangan-(II)-acetat, Eisen-(II)-acetat, Eisen-(III)-acetat, Eisenoxalat, Cobaltchlorid, Cobaltnaphthenat, Nickelchlorid, Nickelnaphthenat sowie deren Mischungen. Die Katalysatoren können ggf. auch in Form ihrer Hydrate oder Ammoniakate zu Einsatz kommen.
   Ausgangsverbindungen für das erfindungsgemäße Verfahren sind cycloaliphatische Diamine der bereits obengenannten Formel (II), Alkohole der bereits obengenannten Formel (III) sowie Harnstoff oder als Carboxylierungsmittel geeignete Harnstoffderivate in Abwesenheit oder Gegenwart von Dialkylcarbonaten, Carbamidsäurealkylestern oder Mischungen aus Dialkylcarbonaten und Carbamidsäurealkylestern.
   Geeignete Diamine der Formel (II) sind beispielsweise 1,4-Diaminocyclohexan, 4,4'-Methylendicyclohexyldiamin, 2,4-Methylendicyclohexyldiamin, 2,2'-Methylendicyclohexyldiamin und isomere cycloaliphatische Diamine sowie perhydriertes Methylendiphenyldiamin. Methylendiphenyldiamin (MDA) fällt herstellungsbedingt als Isomerenmischung aus 4,4'-, 2,4- und 2,2'-MDA an (s. z. B. DE 101 27 273). Perhydriertes Methylendiphenyldiamin wird durch vollständige Hydrierung von MDA erhalten und ist demzufolge eine Mischung aus isomeren Methylendicyclohexyldiaminen (H₁₂MDA), nämlich 4,4'-, 2,4- und 2,2'-H₁₂MDA. Bevorzugt werden als Diamine der Formel (II) 4,4'-Methylendicyclohexyldiamin, 2,4-Methylendicyclohexyldiamin und 2,2'-Methylendicyclohexyl-diamin sowie auch beliebige Mischungen mindestens zweier dieser Isomere eingesetzt. Selbstverständlich können auch Diamine zum Einsatz gelangen, die von der Formel (II) abweichen. Beispielhaft seien 1,3- und 1,4-Diaminomethylcyclohexan und 3-Aminomethyl-3,5,5-trimethylcyclohexylamin aufgeführt. Der Einsatz von Aminen, die von der Formel (II) abweichen, ist jedoch nicht bevorzugt.
   Als Alkohole der Formel (III) eignen sich beliebige aliphatische oder cycloaliphatische Alkohole, die unter Normaldruck einen unterhalb 190 °C liegenden Siedepunkt aufweisen. Beispielhaft genannt seinen C1-C6-Alkanole wie z. B. Methanol, Ethanol, 1-Propanol, 1-Butanol, 2-Butanol, 1-Hexanol oder Cyclohexanol. Bevorzugt wird 1-Butanol als Alkohol verwendet.
   Im Zuge der Umsetzung des Reaktionsgemische wird Ammoniak freigesetzt, dessen Entfernung aus dem Reaktionsgleichgewicht sich als vorteilhaft erwiesen hat. Beim Austrag des Ammoniaks aus dem Reaktor ist darauf zu achten, dass die Wandtemperaturen des Reaktors und des Austragsrohres oberhalb von 60 °C liegen, damit eine Belegung durch Ammoniumcarbaminat, welches in minimalen Mengen aus Ammoniak und Kohlendioxid durch Zersetzung von Harnstoff gebildet wird, vermieden werden kann. Es hat sich beispielsweise bewährt, die Umsetzung in einem Druckdestillationsreaktor durchzuführen, wobei das Reaktionsgemisch im Gegenstrom zu im Sumpf eingebrachten Alkoholbrüden gerührt wird und auf diese Weise eine derartig intensive Durchmischung der Flüssigkeit auf den Böden, dass sie praktisch jeweils einer Kaskadenstufe entsprechen, erfolgt. Das am Kopf abgezogene, dampfförmige Gemisch aus Alkohol und Ammoniak kann, vorzugsweise unter dem Druck des Druckdestillationsreaktors und ohne es vorher zu kondensieren, in eine Destillationskolonne geführt werden, vom Ammoniak freien Alkohol zu gewinnen, der in den Sumpf des Druckdestillationsreaktors und der Kolonne zurückgeführt wird. Um eine Belegung des Rückflusskondensators mit Ammoniumcarbaminat zu verhindern, lässt man in diesem zur Einstellung der Temperatur am Kopf auf mindestens 60 °C einen entsprechenden Anteil an Alkohol zu.
b) Der überschüssige Alkohol, die Dialkylcarbonate, sofern solche gebildet wurden oder in der Reaktionsmischung vorliegen, oder Carbamidsäurealkylester oder Mischungen aus mindestens zwei dieser Komponenten werden vorteilhafterweise zweistufig abgetrennt. Auf der ersten Stufe wird die Reaktionsmischung vom Druckniveau der Reaktionsstufe a) auf einen Druck von 1 - 500 mbar, vorzugsweise 2 - 150 mbar, entspannt und auf diese Weise in gasförmige Brüden, die die überwiegende Alkoholmenge sowie ggf. Dialkylcarbonate und/oder Carbamidsäurealkylester enthalten, und in einen flüssigen Austrag aufgetrennt. Im zweiten Schritt wird der flüssige Austrag durch Dünnschichtverdampfung bei 180 °C - 250 °C, vorzugsweise 200 °C - 230 °C, und einem Druck von 0,1 mbar bis 20 mbar, vorzugsweise 1 mbar bis 10 mbar, von ggf. vorhandenem restlichen Alkohol sowie Mittelsiedern wie Dialkylcarbonaten und/oder Carbamidsäurealkylestern befreit, so dass der Rückstand im wesentlichen aus dem monomeren Diurethan und gegebenenfalls hochsiedenden Oligomeren besteht.
   Die Brüden können, vorzugsweise nach destillativer Reinigung, optional in die Reaktionsstufe a) zurückgeführt werden.
c) Der nach Abtrennung der Brüden aus Stufe b) erhaltene flüssige, die monomeren Diurethane und gegebenenfalls hochsiedende Oligomere enthaltende Stoffstrom wird destillativ, vorzugsweise mit Hilfe eines Dünnschicht- oder Kurzwegverdampfers, bei einer Temperatur von 180 - 260 °C, vorzugsweise 200 - 240 °C und unter einem Druck von 0,01 - 10 mbar, vorzugsweise 0,02 - 5 mbar in einen Wertstoffstrom, der die monomeren Diurethane und die leichter siedenden Nebenprodukte enthält, und einen nicht destillierbaren Nebenproduktstrom getrennt, der aus dem Herstellverfahren ausgeschleust und üblicherweise als stofflich nicht verwertbarer Rückstand verworfen wird.
   Optional kann der gegebenenfalls hochsiedende Oligomere enthaltende Stoffstrom aus Stufe b) vor seiner oben beschriebenen destillativen Aufreinigung auch in zwei Teilströme aufgeteilt werden, von denen einer direkt der Spaltungsreaktion (Stufe d)) zugeführt wird und der andere zunächst die oben beschriebene Hochsiederabtrennung durchläuft.
d) Der die monomeren Diurethane und die leichter siedenden Nebenprodukte enthaltende Wertstoffstrom wird in einer geeigneten Vorrichtung teilweise, lösemittelfrei in flüssiger Phase in Gegenwart von Katalysatoren bei Temperaturen von 180 - 280 °C, vorzugsweise 200 - 260 °C, und unter einem Druck von 0,1 - 200 mbar, vorzugsweise 0,2 - 100 mbar kontinuierlich thermisch gespalten. Der Umsatz von Diurethan zu Diisocyanat in der Vorrichtung zur thermischen Spaltung kann in Abhängigkeit vom verwendeten Polyurethan weitgehend frei gewählt werden und liegt üblicherweise in einem Bereich von 10 - 95 Gew.%, vorzugsweise 35-85 % der zugeführten Diurethanmenge (Feed). Der ungespaltene Anteil der Reaktionsmischung, der nicht umgesetzte Diurethane, hochsiedende Nebenprodukte und andere wieder verwertbare und unverwertbare Nebenprodukte enthält, wird kontinuierlich ausgeschleust. Die Menge der Ausschleusung richtet sich u.a. nach dem gewünschten Umsatz und der gewünschten Kapazität der Spaltreaktion und kann leicht experimentell ermittelt werden. Sie beträgt üblicherweise 10 - 60 Gew.-%, vorzugsweise 15 - 45 Gew.-%, bezogen auf den Feed.
   Als Katalysatoren zur chemischen Spaltung der Diurethane finden z. B. die vorgenannten, die Urethanbildung katalysierenden anorganischen und organischen Verbindungen Verwendung. Vorzugsweise werden Chloride des Zinks oder Zinns sowie Zink-, Mangan-, Eisen-, oder Cobaltoxide eingesetzt, wobei der Katalysator dem Wertstoffstrom aus der Reinigungsstufe c) vor deren Zuführung in die Spaltung als 0,01 - 25 Gew.%ige, vorzugsweise 0,05 - 10 Gew.%ige Lösung oder Suspension in dem Alkohol, der auch zur Urethanherstellung verwendet wird, in einer Menge von 5 - 400 ppm, vorzugsweise 10 - 100 ppm, zudosiert wird.
   Als Spaltvorrichtungen eigenen sich beispielsweise zylinderförmige Spaltreaktoren, wie z. B. Röhrenöfen oder vorzugsweise Verdampfer, beispielsweise Fallfilm-, Dünnschicht- oder Bulkverdampfer, wie z. B. Robertverdampfer, Herbertverdampfer, caddle-typ-Verdampfer, Oskarverdampfer und Heizkerzenverdampfer.
   Prinzipiell geht es darum, die mittlere Verweilzeit der Isocyanatgruppen, die bei Deblockierung des Alkohols zwangsläufig freigesetzt werden, in der Spaltzone möglichst gering zu halten und so unerwünschte Nebenreaktionen auf ein Minimum zu beschränken.
   Bevorzugt wird die Spaltung in einer kombinierten Spalt- und Rektifizierkolonne durchgeführt, die für die Energiezufuhr im Sumpf mit einem Fallfilmverdampfer, im unteren Drittel mit einer Einrichtung zum zusätzlichen Energieeintrag bzw. zur Energierückgewinnung, im oberen Drittel mit einer Einrichtung zum Abzug von Roh-Diisocyanat und am Kopf mit einem Kondensator für den Rückfluss und den Abzug von reinem Alkohol ausgestattet ist.
e) Die bei der thermischen Spaltung in Stufe d) gebildeten Spaltprodukte, die sich vor allem aus Alkohol, Diisocyanat und partiell gespaltenen Diurethanen zusammensetzen, werden durch Rektifikation bei Temperaturen von 95 - 260 °C, vorzugsweise 110 - 245 °C und einem Druck von 0,5 - 250 mbar, vorzugsweise 1 - 100 mbar, in Alkohol und in eine rohe Fraktion, bestehend aus cycloaliphatischem Diisocyanat, partiell gespaltenem cycloaliphatischem Diisocyanat und ggf. geringen Anteilen an cycloaliphatischen Diurethan, getrennt. Diese Trennung kann beispielsweise in der Spaltkolonne der obengenannten kombinierten Spalt- und Rektifizierkolonne (Stufe d) durchgeführt werden.
f) Die vorzugsweise durch Rektifikation in Stufe e) erhaltene rohe Fraktion, bestehend aus cycloaliphatischem Diisocyanat, partiell gespaltenem cycloaliphatischem Diurethan und ggf. geringen Anteilen an cycloaliphatischen Diurethan, wird durch Destillation bei einer Temperatur von 95 - 260 °C, vorzugsweise 110 - 245 °C und unter einem Druck von 0,5 - 150 mbar, vorzugsweise 1 - 75 mbar, gereinigt, wobei die anfallenden Fraktionen in Stufe g) zurückgeführt oder als Reinprodukt isoliert werden.
g) Die Sumpfausschleusung aus der Spaltungsstufe d) wird mit dem Alkohol aus der Rektifikationsstufe e) zusammengeführt, wobei das Molverhältnis von NCO-Gruppen und OH-Gruppen bis zu 1 : 100, vorzugsweise 1 : 20 und besonders bevorzugt 1: 10 beträgt, und die Reaktionsmischung in Gegenwart oder Abwesenheit von Katalysatoren innerhalb von 1 - 150 min, vorzugsweise 3 - 60 min, bei Temperaturen von 20 - 200 °C, vorzugsweise 50 - 170 °C und bei einem Druck von 0,5 - 20 bar, vorzugsweise 1 - 15 bar, umgesetzt. Die Umsetzung kann in einer kontinuierlichen Kesselkaskade oder in einem Rohrreaktor durchgeführt werden.
   Als Katalysatoren kommen grundsätzlich alle Kontakte in Frage, die die NCO/OH-Reaktion fördern. Beispielhaft seien Zinnoctoat, Dibutylzinnlaurat, Zinndichlorid, Zinkdichlorid und Triethylamin aufgeführt.
h) Ein Teil der Sumpffraktion der Reindestillation f) wird kontinuierlich ausgeschleust und optional in die Spaltungsstufe d) oder in die Urethanisierungsstufe g) zurückgeführt. Die Rückführung in die Urethanisierungsstufe ist bevorzugt. Die Menge der Ausschleusung beträgt 0,1 - 50 Gew.%, vorzugsweise 0,2 - 25 Gew.% des Zulaufs an rohem Diisocyanat in die Reindestillationsstufe.
i) Die Kopffraktion der Reindestillationstufe f) kann vorworfen oder vorzugsweise in die Urethanisierungsstufe g) zurückgeführt werden. Die Menge der pro Zeiteinheit abgeführten Kopffraktion beträgt 0,1 - 3 Gew.%, vorzugsweise 0,3 - 1 Gew.% des Zulaufs an rohem Diisocyanat in die Reindestillation.
j) Der Stoffstrom aus der Urethanisierungsstufe g) wird in die Leicht- und Mittelsiederabtrennung b) zurückgeführt.
k) Alternativ zur unter j) beschriebenen Rückführung kann der Stoffstrom aus der Urethanisierungsstufe g) auch in die Diurethanherstellung a) zurückgeführt werden, sofern die Urethanisierung in Gegenwart spezieller Lewis-Säure-Katalysatoren durchgeführt wurde. Unter speziellen Katalysatoren werden in diesem Zusammenhang Halogenide von Fe(III) oder Cu(I) oder Mischungen davon verstanden. Beispielhaft seinen Fe(III)-chlorid, Fe(III)-bromid, Cu(I)-chlorid und Cu(I)-bromid aufgeführt. Der Einsatz dieser speziellen Katalysatoren schließt die gleichzeitige Verwendung anderer Katalysatoren, die der Beschleunigung der Urethanisierung dienen, nicht grundsätzlich aus. Bevorzugt werden die speziellen Katalysatoren, d.h. die Halogenide von Fe(III) oder Cu(I) oder Mischungen davon, ohne zusätzliche Verwendung weiterer Kontakte eingesetzt.

Mit dem erfindungsgemäßen mehrstufigen Verfahren zur kontinuierlichen Herstellung von cycloaliphatischen Diisocyanaten unter Rückführung und Ausschleusung der Nebenprodukte kann für destillierbare cycloaliphatische Polyisocyanate, vorzugsweise cycloaliphatische Diisocyanate, über einen langen Zeitraum eine mit hoher Selektivität störungsfrei ablaufende Reaktion gewährleistet werden. Das erfindungsgemäße Verfahren eignet sich insbesondere zur Herstellung von cycloaliphatischen Diisocyanaten mit 4 bis 18, vorzugsweise 5 bis 15 Kohlenstoffatomen wie 1,4-Diisocyantocyclohexan, 4,4'-Methylendicyclohexyldiisocyanat (4,4'-H₁₂MDI), 2,2'-Methylendicyclohexyldiisocyanat (2,2'-H₁₂MDI), 2,4-Methylendicyclohexyldiisocyanat (2,4-H₁₂MDI) oder auch Mischungen der vorgenannten isomeren Methylendicyclohexyldiisocyanate (H₁₂MDI), wie sie zum Beispiel naturgemäß bei der Umwandlung von perhydriertem MDA in H₁₂MDI anfallen.

Die hergestellten cycloaliphatischen Diisocyanate eigenen sich bestens zur Herstellung von Urethan-, Isocyanurat-, Amid- und/oder Harnstoffgruppen enthaltenden Kunststoffen nach dem Polyisocyanat-Polyadditionsverfahren. Sie finden darüber hinaus Verwendung zur Herstellung von mit Urethan-, Biuret-, und/oder Isocyanuratgruppen modifizierten Polyisocyanatmischungen. Derartige Polyisocyanatmischungen aus cycloaliphatischen Diisocyanaten werden insbesondere zur Herstellung von hochwertigen, lichtbeständigen Polyurethanbeschichtungen eingesetzt.

### Experimenteller Teil

Die Mengenangaben sind Mittelwerte, die nach 10 - 12 stündigem stationärem Betrieb des jeweiligen Kreislaufexperiments erhalten wurden.

### Beispiel 1: Erfindungsgemässe Herstellung von Methylendicyclohexyldiisocyanat (H₁₂MDI) aus perhydriertem Methylendiphenyldiamin (H₁₂MDA) und Harnstoff in Gegenwart von n-Butanol - Rückführung des Diurethanisats in die Flash-Stufe.

Der oberste Boden eines Druckdestillationsreaktors wurden stündlich mit 255,3 g H₁₂MDA, 149,3 g Harnstoff und 545 g n-Butanol beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugeführt. Die verbleibenden 605,9 g/h Bis-(4-butoxycarbonylamino-cyclohexyl)-methan (H₁₂MDU) wurden als Schmelze (140 °C) in die Wälzung des Fallfilmverdampfers der Spalt- und Rektifizierkolonne gefahren, wobei die Deblockierungsreaktion bei einer Temperatur von 236 °C und einem Sumpfdruck von 10 mbar in Gegenwart einer stationären Konzentration an Zinndichlorid von 16 ppm durchgeführt wurde. Die Spaltgase H₁₂MDI und Butanol wurden in zwei hintereinandergeschalteten Kondensatoren bei 85 °C und -25 °C auskondensiert. Das gewonnene, etwa 97 %ige Roh- H₁₂MDI wurde einer Reindestillation zugeführt, wobei 270,33 g/h H₁₂MDI mit einer Reinheit von > 99,5 % erhalten wurden, was einer Selektivität von 85 % entspricht. 177,2 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 147,6 g/h aus dem Wälzkreislauf ausgeschleust und mit 24,0 g/h Sumpfauskreisung aus der H₁₂MDI-Reindestillation sowie dem Kopfprodukt aus der Spalt- und Rektifizierkolonne vereinigt und reurethanisiert. Das Reurethanisat wurde zusammen mit dem Reaktoraustrag der Diurethanherstellung dem Flash-Behälter zugeführt.

### Beispiel 2: Erfindungsgemäße Herstellung von Methylendicyclohexyldiisocyanat (H₁₂MDI) aus perhydriertem Methylendiphenyldiamin (H₁₂MDA) und Harnstoff in Gegenwart von n-Butanol - Reurethanisierung in Gegenwart von CuCl und Rückführung des Reurethanisats in die Diurethansynthese.

Der oberste Boden eines Druckdestillationsreaktors wurden stündlich mit 255,3 g H₁₂MDA, 149,3 g Harnstoff und 545 g n-Butanol sowie dem Stoffstrom aus der katalytischen Reurethanisierung beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde, zusammen mit dem Stoffstrom aus der Reurethanisierung, im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugeführt. Die verbleibenden 601,1 g/h Bis-(4-butoxycarbonylamino-cyclohexyl)-methan (H₁₂MDU) wurden als Schmelze (140 °C) in die Wälzung des Fallfilmverdampfers der Spalt- und Rektifizierkolonne gefahren, wobei die Deblockierungsreaktion bei einer Temperatur von 236°C und einem Sumpfdruck von 10 mbar in Gegenwart einer stationären Konzentration an Zinndichlorid von 16 ppm durchgeführt wurde. Die Spaltgase H₁₂MDI und Butanol wurden in zwei hintereinandergeschalteten Kondensatoren bei 85 °C und -25 °C auskondensiert. Das gewonnene, etwa 97 %ige Roh- H₁₂MDI wurde einer Reindestillation zugeführt, wobei 268,2 g/h H₁₂MDI mit einer Reinheit von > 99,5 % erhalten wurden, was einer Selektivität von 84 % entspricht. 175,9 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 145,6 g/h aus dem Wälzkreislauf ausgeschleust und mit 23,9 g/h Sumpfauskreisung aus der H₁₂MDI-Reindestillation sowie dem Kopfprodukt aus der Spalt- und Rektifizier-kolonne vereinigt und in Gegenwart von 100 ppm CuCl reurethanisiert. Das Reurethanisat wurde der Diurethanherstellung im Druckdestillationsreaktor zugeführt.

### Vergleichsbeispiel 1: Herstellung von Methylendicyclohexyldiisocyanat (H₁₂MDI) aus perhydriertem Methylendiphenyldiamin (H₁₂MDA) und Harnstoff in Gegenwart von n-Butanol - Reurethanisierung und Rückführung des Reurethanisats in die Diurethansynthese.

Der oberste Boden eines Druckdestillationsreaktors wurden stündlich mit 255,3 g H₁₂MDA, 149,3 g Harnstoff und 545 g n-Butanol sowie dem Stoffstrom aus der Reurethanisierung beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugeführt. Die verbleibenden 575,1 g/h Bis-(4-butoxycarbonylamino-cyclohexyl)-methan (H₁₂MDU) wurden als Schmelze (140 °C) in die Wälzung des Fallfilmverdampfers der Spalt- und Rektifizierkolonne gefahren, wobei die Deblockierungsreaktion bei einer Temperatur von 236°C und einem Sumpfdruck von 10 mbar in Gegenwart einer stationären Konzentration an Zinndichlorid von 16 ppm durchgeführt wurde. Die Spaltgase H₁₂MDI und Butanol wurden in zwei hintereinandergeschalteten Kondensatoren bei 85 °C und -25 °C auskondensiert. Das gewonnene, etwa 97 %ige Roh- H₁₂MDI wurde einer Reindestillation zugeführt, wobei 252,9 g/h H₁₂MDI mit einer Reinheit von > 99,5 % erhalten wurden, was einer Selektivität von 79 % entspricht. 163,5 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 139,9 g/h aus dem Wälzkreislauf ausgeschleust und mit 22,6 g/h Sumpfauskreisung aus der H₁₂MDI-Reindestillation sowie dem Kopfprodukt aus der Spalt- und Rektifizierkolonne vereinigt und reurethanisiert. Das Reurethanisat wurde der Diurethanherstellung im Druckdestillationsreaktor zugeführt.

Die Anfangsselektivität des Kreislaufversuchs lag bei ca. 84 %. Sie nahm im Verlauf des Experiments (12 h) jedoch kontinuierlich ab und fiel am Ende unter 75 %.

### Vergleichsbeispiel 2: Herstellung von Methylendicyclohexyldiisocyanat (H₁₂MDI) aus perhydriertem Methylendiphenyldiamin (H₁₂MDA) und Harnstoff in Gegenwart von n-Butanol - direkte Rückführung der Spaltausschleusung in die Diurethansynthese.

Der oberste Boden eines Druckdestillationsreaktors wurden stündlich mit 255,3 g H₁₂MDA, 149,3 g Harnstoff und 545 g n-Butanol sowie dem Stoffstrom aus der Spaltreaktorausschleusung und dem Kopfprodukt der Spalt- und Rektifizierkolonne (Butanol) beaufschlagt und die Reaktionsmischung unter kontinuierlicher Entfernung des freigesetzten Ammoniaks bei 11 - 14 bar, 220 °C und einer mittleren Verweilzeit von 8,5 h gekocht. Der Reaktoraustrag wurde im Flash-Behälter bei 55 mbar und anschließender Dünnschichtverdampfung bei 220 °C und 2 mbar von überschüssigem Alkohol, Leicht- und Mittelsiedern befreit und der Hochsiederabtrennung durch Kurzwegverdampfung bei 0,08 mbar zugeführt. Die verbleibenden 571,8 g/h Bis-(4-butoxycarbonylamino-cyclohexyl)-methan (H₁₂MDU) wurden als Schmelze (140 °C) in die Wälzung des Fallfilmverdampfers der Spalt- und Rektifizierkolonne gefahren, wobei die Deblockierungsreaktion bei einer Temperatur von 236 °C und einem Sumpfdruck von 10 mbar in Gegenwart einer stationären Konzentration an Zinndichlorid von 16 ppm durchgeführt wurde. Die Spaltgase H₁₂MDI und Butanol wurden in zwei hintereinandergeschalteten Kondensatoren bei 85 °C und -25 °C auskondensiert. Das gewonnene, etwa 97 %ige Roh- H₁₂MDI wurde einer Reindestillation zugeführt, wobei 249,8 g/h H₁₂MDI mit einer Reinheit von > 99,5 % erhalten wurden, was einer Selektivität von 78 % entspricht. 160,6 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurden 137,5 g/h aus dem Wälzkreislauf ausgeschleust und ohne Reurethanisierung der Diurethanherstellung im Druckdestillationsreaktor zugeführt. 22,8 g/h Sumpfauskreisung aus der H₁₂MDI-Reindestillation wurden in die Wälzung der Spaltapparartur zurückgeführt.

Die Anfangsselektivität des Kreislaufversuchs lag bei ca. 83 %. Sie nahm im Verlauf des Experiments (12 h) jedoch kontinuierlich ab und fiel am Ende unter 75 %.

## Patentansprüche

1. Mehrstufiges Verfahren zur kontinuierlichen Herstellung von cycloaliphatischen Diisocyanaten, durch Umsetzung von cycloalipahtischen Diaminen mit Kohlensäurederivaten und Alkoholen zu cycloaliphatischen Diurethanen und anschließenden thermischen Spaltung der Diurethane zu cycloaliphatischen Diisocyanten,
**dadurch gekennzeichnet,**
**dass** die Diurethane nach ihrer Synthese durch Umsetzung von cycloaliphatischen Diaminen mit Alkohol und Harnstoff und/oder Harnstoff-Derivaten von Leicht-, Mittel- und Hochsiedern befreit werden, das so aufgereinigte cycloaliphatische Diurethan unter Freisetzung des gewünschten cycloaliphatischen Diisocyanats thermisch gespalten wird, ein Teil des Spaltsumpfes der Spaltapparatur kontinuierlich ausgeschleust und mit Alkohol reurethanisiert wird und die Rückführung des Reurethanisatstroms direkt in die Leichtsiederabtrennung erfolgt.

2. Mehrstufiges Verfahren zur kontinuierlichen Herstellung von cycloaliphatischen Diisocyanaten der Formel (I)
OCN-R-NCO
wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Harnstoff und/oder Harstoffderivaten und Alkoholen in cycloaliphatischen Diurethanen, und deren thermische Spaltung, wobei
a) cycloaliphatische Diamine der Formel (II)
H₂N-R-NH₂
wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Stickstoffatome direkt an einem Kohlenwasserstoffcyclus gebunden und zwischen ihnen mindestens 3 Kohlenstoffatome angeordnet sind, mit Harnstoff und/oder Harnstoffderivaten und Alkoholen der Formel (III)
R¹-OH
wobei R¹ für einen Rest steht, wie er nach Entfernung der Hydroxylgruppe aus einem primären oder sekundären (cyclo)aliphatischen Alkohol mit 3 bis 8 Kohlenstoffatomen verbeleibt, in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren zu cycloaliphatischen Diurethanen, umgesetzt werden und das entstehende Ammoniak gleichzeitig abtrennt wird;
b) aus der erhaltenen Reaktionsmischung der Alkohol, die Dialkylcarbonate und/oder Carbamidsäurealkylester abtrennt werden und der Alkohol sowie optional die Dialkylcarbonate und/oder Carbamidsäurealkylester in die Reaktionsstufe a) zurückführt werden;
c) der im wesentlichen Diurethane enthaltende Stoffstrom aus Stufe b) destillativ in einen Wertstoffstrom und einen Nebenproduktstrom, der ausgeschleust wird, getrennt wird;
d) die über die Schritte b) und c) aufgereinigten Diurethane enthaltende Reaktionsmischung in Gegenwart eines Katalysators lösemittelfrei bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig ausgeschleust wird;
e) die Spaltprodukte durch Rektifikation in ein rohes cycloaliphatisches Diisocyanat und Alkohol getrennt werden;
f) das rohe cycloaliphatische Diisocyanat, durch Destillation gereinigt und die Reinproduktfraktion isoliert wird;
g) die Sumpfausschleusung aus d) mit dem Alkohol aus e) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt;
h) ein Teil der Sumpffraktion der Reindestillation f) kontinuierlich ausschleust und in die Spaltreaktion d), vorzugsweise aber die Urethanisierungsstufe g) geführt wird;
i) die bei der Reindestillation des cycloaliphatischen Diisocyanats anfallende Kopffraktion ebenfalls in die Urethanisierungsstufe g) zurückgeführt oder verworfen wird;
j) der Reurethanisatstrom aus g) in Stufe b) zurückgeführt wird;
oder
k) der Reurethanisatstrom aus g) in die Reaktionsstufe a) zurückgeführt wird, unter der Voraussetzung, das g) in Gegenwart von Katalysatoren bevorzugt ausgewählt aus den Halogeniden von Fe(III) und/oder Cu(I), durchgeführt wird.

3. Mehrstufiges Verfahren nach Anspruch 1 oder 2,
**dadurch gekennzeichnet**,
das als cycloaliphatisches Diamin 4,4'-Methylendicyclohexyldiamin, 2,4-Methylendicyclohexyldiamin und 2,2'-Methylendicyclohexyldiamin sowie auch beliebige Mischungen mindestens zweier dieser Isomere eingesetzt werden.

4. Mehrstufiges Verfahren nach Anspruch 1 oder 2,
**dadurch gekennzeichnet,**
**dass** als cycloaliphatisches Diamin 4,4'-Methylendicyclohexyldiamin und/oder isomere cycloaliphatische Diamine eingesetzt werden.

5. Mehrstufiges Verfahren nach Anspruch 1 oder 2,
**dadurch gekennzeichnet,**
**dass** als cycloaliphatisches Diamin 1,4-Diaminocyclohexan eingesetzt wird.

6. Mehrstufiges Verfahren nach mindestens einem der vorherigen Ansprüche,
**dadurch gekennzeichnet,**
**dass** die Stufe a) kontinuierlich in einem Destillationsreaktor oder in einer Rührkesselkaskade durchgeführt wird.

7. Mehrstufiges Verfahren nach mindestens einem der vorherigen Ansprüche,
**dadurch gekennzeichnet,**
**dass** die Umsetzung in Stufe a) in einem Molverhältnis von Diamin : Harnstoff : Alkohol von 1 : 2,01 : 4,0 bis 1 : 2, 2 : 10 erfolgt.

8. Mehrstufiges Verfahren nach mindestens einem der vorherigen Ansprüche,
wobei die Verweilzeit der Edukte in Stufe a) 2 bis 20, vorzugsweise 4 bis 9 Stunden beträgt.

9. Mehrstufiges Verfahren nach mindestens einem der vorherigen Ansprüche,
**dadurch gekennzeichnet,**
**dass** die Stufe a) in einem Reaktor bei 140 bis 270 °C und einem Druck von 2 bis 80 bar durchgeführt wird.

10. Mehrstufiges Verfahren nach mindestens einem der vorherigen Ansprüche,
**dadurch gekennzeichnet,**
**dass** in Stufe a) bei Reaktionstemperaturen von 160 bis 250 °C und bei einem Druck von 7 bis 15 bar umgesetzt wird.

11. Mehrstufiges Verfahren nach mindestens einem der vorherigen Ansprüche,
**dadurch gekennzeichnet,**
**dass** die Stufe a) in einem Druckdestillationsreaktor durchgeführt wird.

12. Mehrstufiges Verfahren nach mindestens einem der vorherigen Ansprüche,
wobei in Stufe a) die Edukte kontinuierlich auf den obersten Boden aufgegeben werden und das freigesetzte Ammoniak unterstützt durch Alkoholbrüden, die im Sumpf des Destillationsreaktor eingeführt werden, ausgetrieben wird.

13. Mehrstufiges Verfahren nach mindestens einem der vorherigen Ansprüche,
**dadurch gekennzeichnet,**
**dass** in Stufe a) Alkohole mit 1 - 6 Kohlenstoffatomen eingesetzt werden.

14. Mehrstufiges Verfahren nach mindestens einem der vorherigen Ansprüche,
**dadurch gekennzeichnet,**
**dass** in Stufe a) Butanol verwendet wird.

15. Mehrstufiges Verfahren nach mindestens einem der vorherigen Ansprüche,
**dadurch gekennzeichnet,**
**dass** die Umsetzung in Stufe a) in Gegenwart von Katalysatoren durchgeführt wird.

16. Mehrstufiges Verfahren nach mindestens einem der vorherigen Ansprüche,
**dadurch gekennzeichnet,**
**dass** die Stufe b) zweistufig durchgeführt wird.

17. Mehrstufiges Verfahren nach Anspruch 16,
**dadurch gekennzeichnet,**
**dass** im ersten Schritt der Stufe b) die Reaktionsmischung vom Druckniveau der Reaktionsstufe a) auf einen Druck von 1 bis 500 mbar, vorzugsweise 2 bis 150 mbar, entspannt wird.

18. Mehrstufiges Verfahren nach den Ansprüchen 16 oder 17,
**dadurch gekennzeichnet,**
**dass** im zweiten Schritt der Stufe b) der flüssige Austrag durch Dünnschichtverdampfung bei 180 °C bis 250 °C, vorzugsweise 200 °C bis 230 °C, und einem Druck von 0,1 mbar bis 20 mbar, vorzugsweise 1 mbar bis 10 mbar, von gegebenenfalls vorhandenem restlichen Alkohol sowie Mittelsiedern wie Dialkylcarbonaten und/oder Carbamidsäurealkylestern befreit wird.

19. Mehrstufiges Verfahren nach mindestens einem der vorhergehenden Ansprüchen,
**dadurch gekennzeichnet,**
**dass** die Brüden der Stufe b) nach weiterer destillativer Reinigung in die Reaktionsstufe a) zugeführt werden.

20. Mehrstufiges Verfahren nach mindestens einem der vorhergehenden Ansprüche,
**dadurch gekennzeichnet,**
**dass** die Trennung in Stufe c) bei einer Temperatur von 180 bis 260 °C, vorzugsweise 200 bis 240 °C und unter einem Druck von 0,01 bis 10 mbar, vorzugsweise 0,02 bis 5 mbar durchgeführt wird.

21. Mehrstufiges Verfahren nach mindestens einem der vorhergehenden Ansprüche,
**dadurch gekennzeichnet,**
**dass** die Stufe c) mit Hilfe eines Dünnschicht- oder Kurzwegverdampfers durchgeführt wird.

22. Mehrstufiges Verfahren nach mindestens einem der vorhergehenden Ansprüche,
**dadurch gekennzeichnet,**
**dass** die Nebenprodukte aus Stufe c) ausgeschleust und verworfen werden.

23. Mehrstufiges Verfahren nach mindestens einem der vorhergehenden Ansprüche,
**dadurch gekennzeichnet,**
**dass** der Stoffstrom aus Stufe b) nicht gemäß Stufe c) verarbeitet wird, sondern vor seiner destillativen Aufreinigung in zwei Teilströmen aufgeteilt wird, von denen ein Teilstrom direkt der Spaltreaktion (Stufe d) zugeführt wird.

24. Mehrstufiges Verfahren nach mindestens einem der vorhergehenden Ansprüche,
**dadurch gekennzeichnet,**
**dass** die Stufe d) in einer kombinierten Spalt- und Rektifizierkolonne durchgeführt wird.

25. Mehrstufiges Verfahren nach mindestens einem der vorhergehenden Ansprüche,
**dadurch gekennzeichnet,**
**dass** in Stufe d) bei Temperaturen von 180 bis 280 °C, vorzugsweise 200 bis 260 °C, und unter einem Druck von 0,1 bis 200 mbar, vorzugsweise 0,2 bis 100 mbar, kontinuierlich thermisch gespalten wird.

26. Mehrstufiges Verfahren nach mindestens einem der vorhergehenden Ansprüche,
**dadurch gekennzeichnet,**
**dass** in Stufe d) lösemittelfrei in flüssiger Phase gespalten wird.

27. Mehrstufiges Verfahren nach mindestens einem der vorhergehenden Ansprüche,
**dadurch gekennzeichnet,**
**dass** die Stufe d) in Gegenwart von Katalysatoren durchgeführt wird.

28. Mehrstufiges Verfahren nach mindestens einem der vorhergehenden Ansprüche,
**dadurch gekennzeichnet,**
**dass** die thermisch induzierte Diurethanspaltung der Stufe d) in Röhrenöfen oder vorzugsweise Verdampfern, wie Fallfilm-, Dünnschicht- oder Bulkverdampfern, ausgewählt aus Robertverdampfern, Herbertverdampfern, caddle-typ-Verdampfern, Oskarverdampfern und Heizkerzenverdampfern, durchgeführt wird.

29. Mehrstufiges Verfahren nach mindestens einem der vorhergehenden Ansprüche,
**dadurch gekennzeichnet,**
**dass** in Stufe d) der Umsatz von Diurethan zu Diisocyanat in Abhängigkeit vom verwendeten Diurethan frei gewählt wird, bevorzugt in einem Bereich von 10 bis 95 Gew.-%, vorzugsweise 35 bis 85 Gew.-% der zugeführten Diurethanmenge (Feed) liegt.

30. Mehrstufiges Verfahren nach mindestens einem der vorhergehenden Ansprüche,
**dadurch gekennzeichnet,**
**dass** in Stufe d) ein Teil der Reaktionsmischung, der nicht umgesetzte Diurethane, hochsiedende Nebenprodukte und andere wieder verwertbare und unverwertbare Nebenprodukte enthält, kontinuierlich ausgeschleust wird.

31. Mehrstufiges Verfahren nach Anspruch 30,
**dadurch gekennzeichnet,**
**dass** die Menge der Ausschleusung 10 bis 60 Gew.-%, vorzugsweise 15 bis 45 Gew.-%, bezogen auf den Feed, beträgt.

32. Mehrstufiges Verfahren nach mindestens einem der vorhergehenden Ansprüche,
**dadurch gekennzeichnet,**
**dass** die Stufe e) in einer kombinierten Spalt- und Rektifizierkolonne durchgeführt wird.

33. Mehrstufiges Verfahren nach mindestens einem der vorhergehenden Ansprüche,
**dadurch gekennzeichnet,**
**dass** in Stufe e) bei Temperaturen von 95 bis 260 °C, vorzugsweise 110 bis 245 °C und einem Druck von 0,5 bis 250 mbar, vorzugsweise 1 bis 100 mbar, verfahren wird.

34. Mehrstufiges Verfahren nach mindestens einem der vorherigen Ansprüche,
**dadurch gekennzeichnet,**
**dass** die aus Stufe e) erhaltene rohe Fraktion, bestehend aus cycloaliphatischen Diisocyanat, partiell gespaltenem cycloaliphatischen Diurethan und gegebenenfalls geringen Anteilen an cyclialiphatischen Diurethan, in Stufe f) durch Destillation bei einer Temperatur von 95 bis 260 °C, vorzugsweise 110 bis 245 °C und unter einem Druck von 0,5 bis 150 mbar, vorzugsweise 1 bis 75 mbar, gereinigt wird.

35. Mehrstufiges Verfahren nach Anspruch 34,
**dadurch gekennzeichnet,**
**dass** die in Stufe f) anfallende Fraktion als Reinprodukt isoliert oder in Stufe **g)** zurückgeführt wird.

36. Mehrstufiges Verfahren nach mindestens einem der vorhergehenden Ansprüche,
**dadurch gekennzeichnet,**
**dass** die Stufe g) in einer kontinuierlichen Kesselkaskade oder in einem Rohrreaktor durchgeführt wird.

37. Mehrstufiges Verfahren nach mindestens einem der vorhergehenden Ansprüche,
**dadurch gekennzeichnet,**
**dass** die Umsetzung in Stufe g) in Gegenwart von Katalysatoren aus der Gruppe der Sn- und/oder Zn-Carboxylate oder -Halogenide und/oder tert. Amine erfolgt.

38. Mehrstufiges Verfahren nach mindestens einem der vorhergehenden Ansprüche,
**dadurch gekennzeichnet,**
**dass** in Stufe h) die Rückführung in die Urethanisierungsstufe g) erfolgt.

39. Mehrstufiges Verfahren nach mindestens einem der vorhergehenden Ansprüche,
**dadurch gekennzeichnet,**
**dass** in Stufe h) die Menge der Ausschleusung 0,1 bis 50 Gew.-%, vorzugsweise 0,2 bis 25 Gew.-% des Zulaufs an rohem Polyisocyanat in die Reindestillationsstufe (Stufe f) beträgt.

40. Mehrstufiges Verfahren nach mindestens einem der vorhergehenden Ansprüche,
**dadurch gekennzeichnet,**
**dass** in Stufe i) die Menge der pro Zeiteinheit abgeführten Kopffraktion 0,1 bis 3 Gew.-%, vorzugsweise 0,3 bis 1 Gew.-% des Zulaufs an rohem Diisocyanat in die Reindestillation (Stufe f) beträgt.

41. Mehrstufiges Verfahren nach mindestens einem der vorhergehenden Ansprüche,
**dadurch gekennzeichnet,**
**dass** in Stufe k) Fe(III)-chlorid, Fe(III)-bromit, Cu(I)-chlorid und Cu(I)-bromid eingesetzt werden.

42. Mehrstufiges Verfahren nach mindestens einem der vorhergehenden Ansprüche,
**dadurch gekennzeichnet,**
**dass** 1,4-Diisocyanatocyclohexan, 4,4'-Methylendicyclohexyldiisocyanat, 2,2'-Methylendicyclohexyldiisocyanat, 2,4'-Methylendicyclohexyldiisocyanat oder auch beliebige Mischungen mindestens zweier isomerer Methylendicyclohexyldiisocyanate hergestellt werden.

43. Verfahren nach mindestens einem der vorherigen Ansprüche, d. g. d. Diamine ausgewählt aus 1,3- und 1,4-Diaminomethylcyclohexan und 3-Aminomethyl-3,5,5-trimethylcyclohexylamin eingesetzt werden.

## Claims

1. Multistage process for continuously preparing cycloaliphatic diisocyanates, by reacting cycloaliphatic diamines with carbonic acid derivatives and alcohols to give cycloaliphatic diurethanes and subsequently thermally cleaving the diurethanes to give cycloaliphatic diisocyanates,
**characterized in that**
the diurethanes, after their synthesis by reacting cycloaliphatic diamines with alcohol and urea and/or urea derivatives, are freed of low, middle and high boilers, the cycloaliphatic diurethane purified in this way is thermally cleaved to release the desired cycloaliphatic diisocyanate, a portion of the cleavage residue from the cleavage apparatus is continuously discharged and reurethanized with alcohol and the reurethanization product is recycled directly into the low-boiler separation.

2. Multistage process for continuously preparing cycloaliphatic diisocyanates of the formula (I)
OCN-R-NCO
where R is a bivalent cycloaliphatic hydrocarbon radical having from 4 to 18, preferably from 5 to 15, carbon atoms, with the proviso that the two isocyanate groups are bonded directly to a hydrocarbon cycle and at least 3 carbon atoms are disposed between them, by reacting cycloaliphatic diamines with urea and/or urea derivatives and alcohols to give cycloaliphatic diurethanes and thermally cleaving them, wherein
a) cycloaliphatic diamines of the formula (II)
H₂N-R-NH₂
where R is a bivalent cycloaliphatic hydrocarbon radical having from 4 to 18, preferably from 5 to 15, carbon atoms, with the proviso that the two nitrogen atoms are bonded directly to one hydrocarbon cycle and at least 3 carbon atoms are disposed between them, are reacted with urea and/or urea derivatives and alcohols of the formula (III)
R¹-OH
where R¹ is a radical as remains after removal of the hydroxyl group from a primary or secondary (cyclo)aliphatic alcohol having from 3 to 8 carbon atoms, in the absence or presence of dialkyl carbonates, alkyl carbamates or mixtures of dialkyl carbonates and carbamic esters and in the absence or presence of catalysts to give cycloaliphatic diurethanes, and the ammonia formed is simultaneously removed;
b) the alcohol, the dialkyl carbonates and/or alkyl carbamates are removed from the resulting reaction mixture, and the alcohol and optionally the dialkyl carbonates and/or alkyl carbamates are recycled into the reaction stage a);
c) the material stream essentially comprising diurethanes from stage b) is separated by distillation into a material-of-value stream and a by-product stream which is discharged;
d) the reaction mixture comprising the diurethanes purified by steps b) and c) is continuously and thermally cleaved in the presence of a catalyst and without solvent, at temperatures of from 180 to 280°C, preferably from 200 to 260°C, and under a pressure of from 0.1 to 200 mbar, preferably from 0.2 to 100 mbar, in such a way that a portion of the reaction mixture of from 10 to 60% by weight based on the feed, preferably from 15 to 45% by weight based on the feed, is constantly discharged;
e) the cleavage products are separated by rectification into a crude cycloaliphatic diisocyanate and alcohol;
f) the crude cycloaliphatic diisocyanate is purified by distillation, and the pure product fraction is isolated;
g) the bottoms discharge from d) is reacted with the alcohol from e) in the presence or absence of catalysts within from 1 to 150 min, preferably from 3 to 60 min, at temperatures of from 20 to 200°C, preferably from 50 to 170°C, and a pressure of from 0.5 to 20 bar, preferably from 1 to 15 bar, at a molar ratio of NCO groups to OH groups of up to 1 : 100, preferably 1 : 20 and more preferably 1 : 10;
h) a portion of the bottoms fraction of the purification by distillation f) is continuously discharged and conducted into the cleavage reaction d), but preferably into the urethanization stage g);
i) the top fraction obtained in the purification by distillation of the cycloaliphatic diisocyanate is likewise recycled into the urethanization stage g) or discarded;
j) the reurethanized stream from g) is recycled into stage b);
or
k) the reurethanized stream from g) is recycled into the reaction stage a), under the condition that g) is carried out in the presence of catalysts which are preferably selected from the halides of Fe(III) and/or Cu(I).

3. Multistage process according to Claim 1 or 2,
**characterized in that**
the cycloaliphatic diamine used is 4,4'-dicyclohexylmethanediamine, 2,4-dicyclohexylmethanediamine and 2,2'-dicyclohexylmethanediamine, and also any mixtures of at least two of these isomers.

4. Multistage process according to Claim 1 or 2,
**characterized in that**
the cycloaliphatic diamine used is 4,4`-dicyclohexylmethanediamine and/or isomeric cycloaliphatic diamines.

5. Multistage process according to Claim 1 or 2,
**characterized in that**
the cycloaliphatic diamine used is 1,4-diaminocyclohexane.

6. Multistage process according to at least one of the preceding claims,
**characterized in that**
stage a) is carried out continuously in a distillation reactor or in a stirred tank battery.

7. Multistage process according to at least one of the preceding claims,
**characterized in that**
the reaction in stage a) is effected in a molar ratio of diamine : urea : alcohol of from 1:2.01:4.0 to 1:2.2:10.

8. Multistage process according to at least one of the preceding claims,
wherein the residence time of the reactants in stage a) is from 2 to 20 hours, preferably from 4 to 9 hours.

9. Multistage process according to at least one of the preceding claims,
**characterized in that**
stage a) is carried out in a reactor at from 140 to 270°C and a pressure of from 2 to 80 bar.

10. Multistage process according to at least one of the preceding claims,
**characterized in that**
conversion is effected in stage a) at reaction temperatures of from 160 to 250°C and at a pressure of from 7 to 15 bar.

11. Multistage process according to at least one of the preceding claims,
**characterized in that**
stage a) is carried out in a pressure distillation reactor.

12. Multistage process according to at least one of the preceding claims,
wherein, in stage a), the reactants are supplied continuously to the uppermost tray and the ammonia released is driven out by alcohol vapors which are introduced into the bottom of the distillation reactor.

13. Multistage process according to at least one of the preceding claims,
**characterized in that**
alcohols having from 1-6 carbon atoms are used in stage a).

14. Multistage process according to at least one of the preceding claims,
**characterized in that**
butanol is used in stage a).

15. Multistage process according to at least one of the preceding claims,
**characterized in that**
the reaction in stage a) is carried out in the presence of catalysts.

16. Multistage process according to at least one of the preceding claims,
**characterized in that**
stage b) is carried out in two stages.

17. Multistage process according to Claim 16,
**characterized in that**,
in the first step of stage b), the reaction mixture is decompressed from the pressure level of reaction stage a) to a pressure of from 1 to 500 mbar, preferably from 2 to 150 mbar.

18. Multistage process according to Claims 16 or 17,
**characterized in that**,
in the second step of stage b), the liquid effluent is freed of any residual alcohol present and also middle boilers such as dialkyl carbonates and/or alkyl carbamates by thin-film evaporation at from 180°C to 250°C, preferably from 200°C to 230°C, and a pressure of from 0.1 mbar to 20 mbar, preferably from 1 mbar to 10 mbar.

19. Multistage process according to at least one of the preceding claims,
**characterized in that**
the vapors of stage b) are fed, after further distillative purification, into reaction stage a).

20. Multistage process according to at least one of the preceding claims,
**characterized in that**
the separation in stage c) is carried out at a temperature of from 180 to 260°C, preferably from 200 to 240°C, and under a pressure of from 0.01 to 10 mbar, preferably from 0.02 to 5 mbar.

21. Multistage process according to at least one of the preceding claims,
**characterized in that**
stage c) is carried out with the aid of a thin-film or short-path evaporator.

22. Multistage process according to at least one of the preceding claims,
**characterized in that**
the by-products from stage c) are discharged and discarded.

23. Multistage process according to at least one of the preceding claims,
**characterized in that**
the stream in stage b) is not processed as per stage c), but rather is divided before its distillative purification into two substreams of which one substream is fed directly to the cleavage reaction (stage d)).

24. Multistage process according to at least one of the preceding claims,
**characterized in that**
stage d) is carried out in a combined cleavage and rectification column.

25. Multistage process according to at least one of the preceding claims,
**characterized in that**,
in stage d), thermal cleavage is effected continuously at temperatures of from 180 to 280°C, preferably from 200 to 260°C, and under a pressure of from 0.1 to 200 mbar, preferably from 0.2 to 100 mbar.

26. Multistage process according to at least one of the preceding claims,
**characterized in that**,
in stage d), cleavage is effected without solvent in the liquid phase.

27. Multistage process according to at least one of the preceding claims,
**characterized in that**
stage d) is carried out in the presence of catalysts.

28. Multistage process according to at least one of the preceding claims,
**characterized in that**
the thermally induced diurethane cleavage of stage d) is carried out in tubular furnaces or preferably evaporators, such as falling-film, thin-film or bulk evaporators, selected from Robert evaporators, Herbert evaporators, Caddle-type evaporators, Oskar evaporators and heating cartridge evaporators.

29. Multistage process according to at least one of the preceding claims,
**characterized in that**,
in stage d), the conversion of diurethane to diisocyanate is selected freely depending on the diurethane used, preferably within the range of from 10 to 95% by weight, preferably from 35 to 85% of the diurethane feed.

30. Multistage process according to at least one of the preceding claims,
**characterized in that**,
in stage d), a portion of the reaction mixture which comprises unconverted diurethanes, high-boiling by-products and other reutilizable and nonutilizable by-products is continuously discharged.

31. Multistage process according to Claim 30,
**characterized in that**
the amount of the discharge is from 10 to 60% by weight, preferably from 15 to 45% by weight, based on the feed.

32. Multistage process according to at least one of the preceding claims,
**characterized in that**
stage e) is carried out in a combined cleavage and rectification column.

33. Multistage process according to at least one of the preceding claims,
**characterized in that**
operation is effected in stage e) at temperatures of from 95 to 260°C, preferably from 110 to 245°C, and a pressure of from 0.5 to 250 mbar, preferably from 1 to 100 mbar.

34. Multistage process according to at least one of the preceding claims,
**characterized in that**
the crude fraction obtained from stage e), consisting of cycloaliphatic diisocyanate, partially cleaved cycloaliphatic diurethane and in some cases small fractions of cycloaliphatic diurethane, is purified in stage f) by distilling at a temperature of from 95 to 260°C, preferably from 110 to 245°C, and under a pressure of from 0.5 to 150 mbar, preferably from 1 to 75 mbar.

35. Multistage process according to Claim 34,
**characterized in that**
the fraction obtained in stage f) is isolated as a pure product or recycled into stage g).

36. Multistage process according to at least one of the preceding claims,
**characterized in that**
stage g) is carried out in a continuous tank battery or in a tubular reactor.

37. Multistage process according to at least one of the preceding claims,
**characterized in that**
the reaction in stage g) is effected in the presence of catalysts from the group of tin and/or zinc carboxylates or halides and/or tertiary amines.

38. Multistage process according to at least one of the preceding claims,
**characterized in that**
the recycling in stage h) is into the urethanization stage g).

39. Multistage process according to at least one of the preceding claims,
**characterized in that**,
in stage h), the amount of the discharge is from 0.1 to 50% by weight, preferably from 0.2 to 25% by weight, of the feed of crude polyisocyanate into the purifying distillation (stage f)).

40. Multistage process according to at least one of the preceding claims,
**characterized in that**
the amount of top fraction removed per unit time in stage i) is from 0.1 to 3% by weight, preferably from 0.3 to 1% by weight, of the feed of crude diisocyanate into the purifying distillation (stage f)).

41. Multistage process according to at least one of the preceding claims,
**characterized in that**
Fe(III) chloride, Fe(III) bromide, Cu(I) chloride and Cu(I) bromide are used in stage k).

42. Multistage process according to at least one of the preceding claims,
**characterized in that**
1,4-diisocyanatocyclohexane, 4,4'-dicyclohexylmethane diisocyanate, 2,2'-dicyclohexylmethane diisocyanate, 2,4'-dicyclohexylmethane diisocyanate or else any mixtures of at least two isomeric dicyclohexylmethane diisocyanates are prepared.

43. Process according to at least one of the preceding claims, **characterized in that** diamines selected from 1,3- and 1,4-diaminomethylcyclohexane and 3-aminomethyl-3,5,5-trimethylcyclohexylamine are used.

## Revendications

1. Procédé en plusieurs étapes pour la fabrication en continu de diisocyanates cycloaliphatiques, réalisée en faisant réagir des diamines cycloaliphatiques avec des dérivés d'acide carbonique et des alcools pour générer des diuréthanes cycloaliphatiques, puis en procédant à un clivage thermique des diuréthanes, ce qui permet d'obtenir des diisocyanates cycloaliphatiques,
**caractérisé en ce que**
on débarrasse les diuréthanes, après que l'on a effectué leur synthèse en faisant réagir des diamines cycloaliphatiques avec un alcool et de l'urée et/ou des dérivés d'urée, de substances à point d'ébullition bas, moyen et élevé, on soumet le diuréthane cycloaliphatique ainsi purifié à un clivage thermique, ce qui libère de la sorte le diisoocyanate cycloaliphatique souhaité, on retire de manière continue une partie du fond de clivage de l'appareil de clivage et on soumet le tout à une ré-uréthanisation avec l'alcool et on recycle le courant de ré-uréthanisation directement à l'étape de séparation des substances à bas point d'ébullition.

2. Procédé en plusieurs étapes pour la fabrication en continu de diisocyanates cycloaliphatiques de formule (I) :
OCN-R-NCO
dans laquelle R représente un radical hydrocarboné cycloaliphatique divalent ayant 4 à 18, de préférence, 5 à 15 atomes de carbone, à condition que les deux groupements isocyanate soient directement reliés à un cycle hydrocarboné et qu'au moins trois atomes de carbone soient aménagés entre eux, réalisée en effectuant la réaction de diamines cycloaliphatiques avec de l'urée et/ou des dérivés d'urée et des alcools de manière à former des diuréthanes cycloaliphatiques, puis en effectuant leur clivage thermique, dans lequel on fait réagir :
a) des diamines cycloaliphatiques de formule (II) :
H₂N-R-NH₂
dans laquelle R représente un radical hydrocarboné cycloaliphatique divalent ayant 4 à 18, de préférence, 5 à 15 atomes de carbone, à condition que les deux atomes d'azote soient directement reliés à un cycle hydrocarboné et qu'au moins trois atomes de carbone soient aménagés entre eux, avec de l'urée et/ou des dérivés d'urée et des alcools de formule (III) :
R¹-OH
dans laquelle R¹ représente un radical, tel qu'il est obtenu après élimination du groupement hydroxyle d'un alcool (cyclo)aliphatique primaire ou secondaire ayant 3 à 8 atomes de carbone, en l'absence ou en présence de dialkylecarbonates, d'esters d'acide carbamique ou de mélanges de dialkylecarbonates et d'esters alkyliques d'acide carbamique, et en l'absence ou en présence de catalyseurs, de manière à obtenir des diuréthanes cycloaliphatiques, et on sépare simultanément l'ammoniac qui se forme,
b) on sépare du mélange réactionnel, l'alcool, les dialkylecarbonates et/ou les esters alkyliques d'acide carbamique et on recycle l'alcool, ainsi que, éventuellement, les dialkylecarbonates et/ou les esters alkyliques d'acide carbamique à l'étape réactionnelle a) ;
c) on sépare par distillation le courant de substances provenant de l'étape b), contenant essentiellement des diuréthanes, ce qui donne un courant de produits intéressants et un courant de produits secondaires que l'on retire ;
d) on soumet le mélange réactionnel contenant des diuréthanes purifiés par le biais des étapes b) et c) à un clivage thermique en mode continu, en présence d'un catalyseur sans solvant, à des températures de 180 à 280 °C, de préférence, de 200 à 260 °C et sous une pression de 0,1 à 200 mbars, de préférence, de 0,2 à 100 mbars, de manière à retirer en permanence une partie du mélange réactionnel représentant 10 à 60 % en poids par rapport à l'alimentation, de préférence, 15 à 45 % en poids par rapport à l'alimentation ;
e) on sépare les produits de clivage en effectuant une rectification qui fournit un diisocyanate cycloaliphatique brut et l'alcool ;
f) on purifie le diisocyanate cycloaliphatique brut par distillation et on isole la fraction de produit pur ;
g) on fait réagir le produit de fond de colonne retiré à l'étape d) avec l'alcool issu de l'étape e) en présence ou en l'absence de catalyseurs, sur une période de 1 à 150 mn, de préférence, de 3 à 60 mn, à des températures de 20 à 200 °C, de préférence, de 50 à 170 °C et sous une pression de 0,5 à 20 bars, de préférence, de 1 à 15 bars, le rapport molaire des groupements NCO aux groupements OH étant de 1:100, de préférence, de 1:20 et, mieux encore, de 1:10
h) on retire en continu une partie de la fraction de fond de colonne de la distillation de purification de l'étape f) et on la délivre à la réaction de clivage de l'étape d), de préférence, à l'étape d'uréthanisation g) ;
i) on recycle la fraction de tête formée lors de la distillation de purification du diisocyanate cycloaliphatique également à l'étape d'uréthanisation g), ou on la met au rebut ;
j) on recycle le courant de ré-uréthanisation obtenu à l'étape g), à l'étape b) ; ou
k) on recycle le courant de ré-uréthanisation obtenu à l'étape g), à l'étape réactionnelle a), à condition que l'étape g) soit réalisée en présence de catalyseurs, de préférence, choisis parmi les halogénures de Fe(III) et/ou de Cu(I).

3. Procédé en plusieurs étapes selon la revendication 1 ou 2,
**caractérisé en ce que**
on utilise, comme diamine cycloaliphatique, la 4,4'-méthylènedicyclohexyldiamine, la 2,4-méthylènedicyclohexyldiamine et la 2,2'-méthylènedicyclohexyldiamine, ainsi que n'importe quel mélange d'au moins deux de ces isomères.

4. Procédé en plusieurs étapes selon la revendication 1 ou 2,
**caractérisé en ce que**
on utilise, comme diamine cycloaliphatique, la 4,4'-méthylènedicyclohexyldiamine et/ou des diamines cycloaliphatiques isomères.

5. Procédé en plusieurs étapes selon la revendication 1 ou 2,
**caractérisé en ce que**
on utilise, comme diamine cycloaliphatique, le 1,4-diaminocyclohexane.

6. Procédé en plusieurs étapes selon l'une quelconque des revendications précédentes,
**caractérisé en ce que**
on réalise l'étape a) en continu dans un réacteur de distillation ou dans des cuves agitées disposées en cascade.

7. Procédé en plusieurs étapes selon l'une quelconque des revendications précédentes,
**caractérisé en ce que**
on effectue la réaction de l'étape a) dans un rapport molaire diamine:urée:alcool de 1:2,01:4,0 à 1:2,2:10.

8. Procédé en plusieurs étapes selon l'une quelconque des revendications précédentes,
dans lequel le temps de séjour des éduits est, à l'étape a), de 2 à 20, de préférence, de 4 à 9 heures.

9. Procédé en plusieurs étapes selon l'une quelconque des revendications précédentes,
**caractérisé en ce que**
on met en oeuvre l'étape a) dans un réacteur à une température de 140 à 270 °C et sous une pression de 2 à 80 bars.

10. Procédé en plusieurs étapes selon l'une quelconque des revendications précédentes,
**caractérisé en ce que**
on met en oeuvre la réaction de l'étape a) à des températures réactionnelles de 160 à 250 °C et sous une pression de 7 à 15 bars.

11. Procédé en plusieurs étapes selon l'une quelconque des revendications précédentes,
**caractérisé en ce que**
on réalise l'étape a) dans un réacteur de distillation sous pression.

12. Procédé en plusieurs étapes selon l'une quelconque des revendications précédentes,
dans lequel, à l'étape a), on délivre les éduits en continu sur le plateau supérieur et on expulse l'ammoniac qui se dégage par le biais de vapeurs d'alcool, lesquelles sont introduites dans le fond du réacteur de distillation.

13. Procédé en plusieurs étapes selon l'une quelconque des revendications précédentes,
**caractérisé en ce que**
on utilise, à l'étape a), des alcools ayant 1 à 6 atomes de carbone.

14. Procédé en plusieurs étapes selon l'une quelconque des revendications précédentes,
**caractérisé en ce que**
on utilise du butanol à l'étape a).

15. Procédé en plusieurs étapes selon l'une quelconque des revendications précédentes,
**caractérisé en ce que**
on réalise la réaction à l'étape a) en présence de catalyseurs.

16. Procédé en plusieurs étapes selon l'une quelconque des revendications précédentes,
**caractérisé en ce que**
on réalise l'étape b) en deux étapes.

17. Procédé en plusieurs étapes selon la revendication 16,
**caractérisé en ce que**
dans la première phase de l'étape b), on détend le mélange réactionnel du niveau de pression appliqué à l'étape réactionnelle a) à une pression de 1 à 500 mbars, de préférence, de 2 à 150 mbars.

18. Procédé en plusieurs étapes selon la revendication 16 ou 17,
**caractérisé en ce que**
dans la deuxième phase de l'étape b), on débarrasse l'extrait liquide, par évaporation en couches minces à une température de 180 °C à 250 °C, de préférence, de 200 °C à 230 °C et sous une pression de 0,1 mbar à 20 mbars, de préférence, de 1 mbar à 10 mbars, de l'alcool résiduel éventuellement présent, de même que de substances à point d'ébullition moyen, telles que les dialkylecarbonates et/ou les esters alkyliques d'acide carbamique.

19. Procédé en plusieurs étapes selon l'une quelconque des revendications précédentes,
**caractérisé en ce que**
les vapeurs de l'étape b) sont acheminées à l'étape réactionnelle a) après une purification supplémentaire par distillation.

20. Procédé en plusieurs étapes selon l'une quelconque des revendications précédentes,
**caractérisé en ce que**
on effectue la séparation à l'étape c) à une température de 180 à 260 °C, de préférence, de 200 à 240 °C et sous une pression de 0,01 à 10 mbars, de préférence, de 0,02 à 5 mbars.

21. Procédé en plusieurs étapes selon l'une quelconque des revendications précédentes,
**caractérisé en ce que**
on réalise l'étape c) à l'aide d'un évaporateur à couche mince ou d'un dispositif d'évaporation éclair.

22. Procédé en plusieurs étapes selon l'une quelconque des revendications précédentes,
**caractérisé en ce que**
les produits secondaires obtenus à l'étape c) sont retirés et mis au rebut.

23. Procédé en plusieurs étapes selon l'une quelconque des revendications précédentes,
**caractérisé en ce que**,
le courant de substances provenant de l'étape b) n'est pas traité selon l'étape c), mais est divisé, avant de procéder à sa purification par distillation, en deux courants partiels, dont l'un d'eux est directement délivré à la réaction de clivage (étape d).

24. Procédé en plusieurs étapes selon l'une quelconque des revendications précédentes,
**caractérisé en ce que**
on réalise l'étape d) dans une colonne de clivage combinée à une colonne de rectification.

25. Procédé en plusieurs étapes selon l'une quelconque des revendications précédentes,
**caractérisé en ce que**
à l'étape d), on effectue le clivage thermique en continu à des températures de 180 à 280 °C, de préférence, de 200 à 260 °C et sous une pression de 0,1 à 200 mbars, de préférence, de 0,2 à 100 mbars.

26. Procédé en plusieurs étapes selon l'une quelconque des revendications précédentes,
**caractérisé en ce que**
à l'étape d), on effectue le clivage en phase liquide en l'absence de solvant.

27. Procédé en plusieurs étapes selon l'une quelconque des revendications précédentes,
**caractérisé en ce que**
on réalise l'étape d) en présence de catalyseurs.

28. Procédé en plusieurs étapes selon l'une quelconque des revendications précédentes,
**caractérisé en ce que**
on effectue le clivage du diuréthane, induit par voie thermique, de l'étape d), dans des fours tubulaires ou, de préférence, dans des évaporateurs, tels que des évaporateurs à film tombant, des évaporateurs à couche mince ou des évaporateurs pour gros volumes, choisis parmi les évaporateurs du type Robert, du type Herbert, du type Caddle, du type Oskar et les évaporateurs à bougies chauffantes.

29. Procédé en plusieurs étapes selon l'une quelconque des revendications précédentes,
**caractérisé en ce que**
à l'étape d), on choisit librement le taux de conversion du diuréthane en diisocyanate, en fonction du diuréthane utilisé, de préférence, dans une plage de 10 à 95 % en poids, de préférence, de 35 à 85 % en poids par rapport à la quantité de diuréthane délivré (alimentation).

30. Procédé en plusieurs étapes selon l'une quelconque des revendications précédentes,
**caractérisé en ce que**
à l'étape d), on retire en continu une partie du mélange réactionnel, qui contient des diuréthanes n'ayant pas réagi, des produits secondaires à point d'ébullition élevé et d'autres produits secondaires exploitables et inexploitables.

31. Procédé en plusieurs étapes selon la revendication 30,
**caractérisé en ce que**
la quantité retirée représente 10 à 60 % en poids, de préférence, 15 à 45 % en poids de l'alimentation.

32. Procédé en plusieurs étapes selon l'une quelconque des revendications précédentes,
**caractérisé en ce que**
on réalise l'étape e) dans une colonne de clivage combinée à une colonne de rectification.

33. Procédé en plusieurs étapes selon l'une quelconque des revendications précédentes,
**caractérisé en ce que**
à l'étape e), on met en oeuvre le procédé à des températures de 95 à 260 °C, de préférence, de 110 à 245 °C et sous une pression de 0,5 à 250 mbars, de préférence, de 1 à 100 mbars.

34. Procédé en plusieurs étapes selon l'une quelconque des revendications précédentes,
**caractérisé en ce que**
la fraction brute obtenue à l'étape e), constituée de diisocyanate cycloaliphatique, de diuréthane cycloaliphatique partiellement clivé et, éventuellement, de faibles fractions de diuréthane cycloaliphatique, est purifiée à l'étape f) par distillation à une température de 95 à 260 °C, de préférence, de 110 à 245 °C, et sous une pression de 0,5 à 150 mbars, de préférence, de 1 à 75 mbars.

35. Procédé en plusieurs étapes selon la revendication 34,
**caractérisé en ce que**
la fraction obtenue à l'étape f) est isolée comme produit pur ou recyclée à l'étape g).

36. Procédé en plusieurs étapes selon l'une quelconque des revendications précédentes,
**caractérisé en ce que**
on réalise l'étape g) en continu dans des cuves disposées en cascade ou dans un réacteur tubulaire.

37. Procédé en plusieurs étapes selon l'une quelconque des revendications précédentes,
**caractérisé en ce que**
on effectue la réaction à l'étape g) en présence de catalyseurs choisis dans le groupe des carboxylates ou des halogénures de Sn et/ou de Zn et/ou de tert-amines.

38. Procédé en plusieurs étapes selon l'une quelconque des revendications précédentes,
**caractérisé en ce que**
à l'étape h), on effectue le recyclage vers l'étape d'uréthanisation g).

39. Procédé en plusieurs étapes selon l'une quelconque des revendications précédentes,
**caractérisé en ce que**
à l'étape h), la quantité retirée représente 0,1 à 50 % en poids, de préférence, 0,2 à 25 % en poids de l'alimentation en polyisocyanate brut de l'étape de distillation de purification (étape f).

40. Procédé en plusieurs étapes selon l'une quelconque des revendications précédentes,
**caractérisé en ce que**
à l'étape i), la quantité de la fraction de tête transportée par unité de temps représente 0,1 à 3 % en poids, de préférence 0,3 à 1 % en poids de l'alimentation en diisocyanate brut de l'étape de distillation de purification (étape f).

41. Procédé en plusieurs étapes selon l'une quelconque des revendications précédentes,
**caractérisé en ce que**
on utilise, à l'étape k), du chlorure de Fe(III), du bromure de Fe(III), du chlorure de Cu(I) et du bromure de Cu(I).

42. Procédé en plusieurs étapes selon l'une quelconque des revendications précédentes,
**caractérisé en ce que**
on fabrique du 1,4-diisocyanatocyclohexane, du 4,4'-méthylènedicyclohexyldiisocyanate, du 2,2'-méthylènedicyclohexyldiisocyanate, du 2,4'-méthylènedicyclohexyldiisocyanate ou, également, n'importe quels mélanges d'au moins deux isomères de méthylènedicyclohexyldiisocyanates.

43. Procédé selon l'une quelconque des revendications précédentes, dans lequel on utilise des diamines choisies parmi le 1,3- et le 1,4-diaminométhylcyclohexane et la 3-aminométhyl-3,5,5-triméthylcyclohexylamine.
